(19) Europäisches Patentamt
European Patent Office
Office européen des brevets

(11) EP 0 502 525 B1

(12) EUROPÄISCHE PATENTSCHRIFT

(45) Veröffentlichungstag und Bekanntmachung des Hinweises auf die Patenterteilung:
26.06.1996 Patentblatt 1996/26

(51) Int Cl.6: **C12P 41/00**
// (C12P41/00, C12R1:38)

(21) Anmeldenummer: 92103780.0

(22) Anmeldetag: 05.03.1992

(54) **Biotechnologisches Verfahren zur Herstellung von S-(+)-2,2-Dimethylcyclopropancarboxamid und R-(-)-2,2-Dimethylcyclopropancarbonsäure**

Biotechnological process for the production of S-(+)-2,2-dimethylcyclopropanecarboxamide and R-(-)-2,2-dimethylcyclopropanecarboxylic acid

Procédé biotechnologique pour la production de S-(+)-2,2-diméthylcyclopropanecarboxamide et acide R-(-)-2,2-diméthylcyclopropanecarboxylique

(84) Benannte Vertragsstaaten:
AT BE CH DE DK ES FR GB IT LI LU NL SE

(30) Priorität: 06.03.1991 CH 678/91
24.06.1991 CH 1854/91

(43) Veröffentlichungstag der Anmeldung:
09.09.1992 Patentblatt 1992/37

(73) Patentinhaber: LONZA AG
CH-3945 Gampel/Wallis (CH)

(72) Erfinder:
• Robins, Karen
Visp (Kanton Wallis) (CH)

• Gilligan, Thomas
Leuk (Kanton Wallis) (CH)

(74) Vertreter: Weinhold, Peter, Dr. et al
Patentanwälte
Dr. Weinhold, Dannenberg,
Dr. Gudel, Schubert
Siegfriedstrasse 8
80803 München (DE)

(56) Entgegenhaltungen:
EP-A- 0 048 301        EP-A- 0 155 779
EP-A- 0 264 457

**Beschreibung**

Die Erfindung betrifft ein neues Verfahren zur Herstellung von optisch aktivem S-(+)-2,2-Dimethylcyclopropancarboxamid und R-(-)-2,2-Dimethylcyclopropancarbonsäure, wobei im racemischen R,S-(±)-2,2-Dimethylcyclopropancarboxamid das R-(-)-2,2-Dimethylcyclopropancarboxamid zur R-(-)-2,2-Dimethylcyclopropancarbonsäure biotransformiert wird und dabei neben R-(-)-2,2-Dimethylcyclopropancarbonsäure S-(+)-2,2-Dimethylcyclopropancarboxamid anfällt.

Im folgenden wird 2,2-Dimethylcyclopropancarboxamid mit 2,2-DMCPCA und 2,2-Dimethylcyclopropancarbonsäure mit 2,2-DMCPCS abgekürzt.

Optisch reines S-(+)-2,2-DMCPCA dient als Ausgangsmaterial zur Herstellung des Dehydropeptidase-Inhibitors Cilastatin, welcher in der Therapie zusammen mit Penem- bzw. Carbapenem-Antibiotika verabreicht wird, um die Desaktivierung der Antibiotika durch eine renale Dehydropeptidase in der Niere zu verhindern (Lit.: EP 048 301).

Chemische Verfahren zur Herstellung von S-(+)-2,2-DMCPCA und 2,2-DMCPCS haben den Nachteil, dass sie aufwendig sind und über mehrere Stufen verlaufen (EP 155779).

Ein mikrobiologisches Verfahren zur Herstellung von S-(+)-2,2-DMCPCS, ausgehend von einem racemishen Estergemisch, ist aus EP 264 457 bekannt.

Die Aufgabe der vorliegenden Erfindung bestand darin, ein biotechnologisches Verfahren zu finden, mit welchem man ausgehend von leicht erhältlichem racemischen R,S-(±)-2,2-DMCPCA in einem Schritt die gewünschten Isomere in hoher Reinheit gewinnen kann.

Diese Aufgabe konnte erfindungsgemäß mit einem neuen Verfahren gemäss Patentanspruch 1 und mit neuen Mikroorganismen gemäss Patentanspruch 10 gelöst werden.

Die erfindungsgemäßen Mikroorganismen sind befähigt, im racemischen R,S-(±)-2,2-DMCPCA das R-(-)-2,2-DMCPCA zur R-(-)-2,2-DMCPCS zu biotransformieren, wobei neben R-(-)-2,2-DMCPCS optisch aktives S-(+)-2,2-DMCPCA anfällt.

Diese Mikroorganismen können beispielsweise aus Bodenproben, Schlamm oder Abwasser unter Zuhilfenahme traditioneller mikrobiologischer Techniken isoliert werden.

Erfindungsgemäss kommen zur Herstellung von S-(+)-2,2-DMCPCA und R-(-)-2,2-DMCPCA alle Mikroorganismen, die R-(-)DMCPCA als Substrat nutzen, in Frage.

Diese Mikroorganismen sind nach folgender Selektionsmethode erhältlich:

a) Mikroorganismen, die mit 2,2-DMCPCA, in Form des Racemats oder seiner optischen Isomere, als N-Quelle und mit einer C-Quelle wachsen, werden auf übliche Weise gezüchtet
b) aus der durch Züchtung erhaltenen Kultur werden dann jene selektioniert, die stabil sind und 2,2-DMCPCA, in Form des Racemats oder seiner optischen Isomere, als einzige N-Quelle nutzen.

Zweckmässig werden die Mikroorganismen selektioniert, die R-(-)-2,2-DMCPCA als N-Quelle akzeptieren.

Als C-Quelle können die Mikroorganismen beispielsweise Zucker, Zuckeralkohole, Carbonsäuren oder Alkohole als Wachstumssubstrat nutzen. Als Zucker kann beispielsweise Fructose oder Glucose Verwendung finden.

Als Zuckeralkohole können beispielsweise Erythrit, Mannit oder Sorbit angewendet werden. Als Carbonsäuren können Mono-, Di- oder Tricarbonsäuren, wie beispielsweise Essigsäure, Malonsäure oder Citronensäure, verwendet werden. Als Alkohole können ein-, zwei- oder dreiwertige Alkohole dienen, wie beispielsweise Ethanol, Glycole oder Glycerin. vorzugsweise wird als C-Quelle ein dreiwertiger Alkohol, wie beispielsweise Glycerin, eingesetzt.

Zweckmässig liegt das C/N-Verhältnis in Selektionsmedium in einem Bereich zwischen 5:1 und 10:1.

Als Selektionsmedium können die in der Fachwelt üblichen angewendet werden, wie beispielsweise ein Mineralsalzmedium, beschrieben in Kulla et al., Arch. Microbiol. 135, S.1-7, 1983. Bevorzugte Mikroorganismen, die mit 2,2-DMCPCA, in Form des Racemats oder seiner optischen Isomere, als N-Quelle und mit einer C-Quelle wachsen, sind:
<u>Comamonas acidovorans</u> A:18 (DSM-Nr.6315), <u>Comamonas acidovorans</u> TG 308 (DSM-Nr.6552), <u>Pseudomonas sp</u> NSAK:42 (DSM-Nr. 6433) und Mikroorganismus <u>Bacterium sp.</u> VIII:II (DSM-Nr.6316) sowie deren Deszendenten und Mutanten.

Die Stämme der DSM-Nr.6315 und 6316 wurden am 29.1.1991, die mit der DSM-Nr.6433 am 25.3.1991 und die mit der DSM-Nr.6552 am 6.4.1991 bei der Deutschen Sammlung von Mikroorganismen und Zellkulturen GmbH, Mascheroder Weg 1B, D-3300 Braunschweig, hinterlegt.

Wissenschaftliche Beschreibung von <u>Comamonas acidovorans A:18</u> (DSM-Nr.6315) :

| Zellform | Stäbchen | Hydrolyse von | |
|---|---|---|---|
| Breite μm | 0,5-0,7 | Stärke | - |
| Länge μm | 1,5-3,0 | Gelatine | - |
| Beweglichkeit | + | Casein | - |
| Geisseln | polar>1 | DNA | - |
| Gram-Reaktion | - | Tween 80 | - |
| Lyse durch 3% KOH | + | Aesculin | - |
| Aminopeptidase (Cerny) | + | Tyrosin-Abbau | - |
| Sporen | - | Substratverwertung | |
| Oxidase | + | Acetat | + |
| Catalase | + | Adipat | + |
| Wachstum | | Caprat | + |
| anaerob | - | Citrat | + |
| 37/41°C | +/- | Glycolat | + |

| | | | |
|---|---|---|---|
| pH 5,6 | + | Lävulinat | + |
| Mac-Conkey-Agar | + | Malat | + |
| SS-Agar | + | Malonat | − |
| Cetrimid-Agar | + | Phenylacetat | + |
| Pigmente | | L-Arabinose | − |
| nicht diffundierend | − | Fructose | + |
| diffundierend | − | Glucose | − |
| fluoreszierend | − | Mannose | − |
| Pyocyanin | − | Maltose | − |
| Säure aus (OF-Test) | | Xylose | − |
| Glucose aerob | − | Inositol | − |
| Glucose anaerob | − | Mannitol | + |
| Gas aus Glucose | − | Gluconat | + |
| Säure aus | | N-Acetylglucosamin | − |
| Glucose | − | L-Serin | − |
| Fructose | + | L-Tryptophan | + |
| Xylose | − | Acetamid | + |
| Mannit | + | Mesaconat | + |
| Glycerol | + | Citraconat | + |
| ONPG | − | L-Tartrat | + |
| ADH | − | N-Quelle | |
| VP | − | $NH_4^+$ | ++ |
| Indol | − | R,S-(±)-2,2-Dimethyl- | |
| $NO_2$ aus $NO_3$ | + | cyclopropanacrboxamid | + |
| Denitrifikation | − | Butyramid | ++ |
| Phenylalanindesaminase | − | Acetamid | + |
| Levan aus Saccharose | − | Propionamid | + |
| Lecithinase | − | Formamid | ± |
| Urease | − | Benzamid | + |
| | | Nicotinamid | + |

API 20NE --> Cs.acidovorans 99,0%

Wissenschaftliche Beschreibung von Comamonas acidovorans TG 308 (DSM-Nr. 6552)

| Zellform | Stäbchen |
|---|---|
| Gram-Reaktion (KOH-Test) | − |

4

(fortgesetzt)

| Zellform | Stäbchen |
|---|---|
| Gram-Färbung | - |
| Sporen | - |
| Beweglichkeit | + |
| °C Wachstum | |
| 37 °C | + |
| 41 °C | - |
| 45 °C | - |
| Katalase | + |
| Oxidase | + |
| Gärung in | |
| Glucose (OF-Test) | - |

Isolate

| | TG308 |
|---|---|
| Nitrat Reduktion | + |
| Indol Produktion | - |
| Säure von Glucose | - |
| Arginindehydrolase | - |
| Urease | - |
| Aesculin Hydrolyse | - |
| Gelatin Hydrolyse | - |
| β-Galactosidase | - |
| Glucose Assimilation | - |
| Arabinose Assimilation | - |
| Mannose Assimilation | - |
| Mannitol Assimilation | + |
| N-Acetyl-glucoseamin Assimilation | - |
| Maltose Assimilation | - |
| Gluconat Assimilation | + |
| Caprat Assimilation | + |
| Adipat Assimilation | + |
| Malat Assimilation | + |
| Citrat Assimilation | - |
| Phenylacetat Assimilation | + |
| Cytochrome Oxidase | + |
| $NO_2$ aus $NO_3$ | + |
| Hydrolyse von Harnstoff | - |
| Verwentung von Fructose | + |
| Alkalisierung von Acetamid | + |
| Alkalisierung von Tartrat | + |
| Alkalisierung von Simmon's Citrat | + |
| Alkalisierung von Malonat | (+) |
| (+) schwach positiv | |

Wissenschaftliche Beschreibung von <u>Pseudomonas sp</u> NSAK:42 (DSM-Nr.6433) :

| | | | |
|---|---|---|---|
| Zellform | Stäbchen | Hydrolyse von | |
| Breite µm | 0,6-0,8 | Stärke | − |
| Länge µm | 1,5-3,0 | Gelatine | − |
| Beweglichkeit | + | Casein | − |
| Gram-Reaktion | − | DNA | − |
| Lyse durch 3% KOH | + | Tween 80 | − |
| Aminopeptidase (Cerny) | + | Aesculin | − |
| Sporen | − | Tyrosin-Abbau | − |
| Oxidase | + | Wachsstoffbedarf | − |
| Catalase | + | Substratverwertung | |
| Wachstum | | Acetat | + |
| anaerob | − | Caprat | + |
| 37/41°C | +/− | Citrat | + |
| pH 5,6 | + | Glycolat | + |
| Mac-Conkey-Agar | + | Lactat | + |
| SS-Agar | − | Lävulinat | + |
| Cetrimid-Agar | − | Malat | + |
| Pigmente | gelb | Malonat | + |
| Säure aus (OF-Test) | | Phenylacetat | + |
| Glucose aerob | − | Suberat | + |
| Glucose anaerob | − | L-Arabinose | − |
| Gas aus Glucose | − | Fructose | + |
| Säure aus | | Glucose | − |
| Glucose | − | Mannose | − |
| Fructose | − | Maltose | − |
| Xylose | − | Xylose | − |
| ONPG | − | Mannitol | − |
| ODC | − | Gluconat | + |
| ADH | − | 2-Ketogluconat | + |
| VP | − | N-Acetylglucosamin | − |
| Indol | − | L-Serin | + |
| $NO_2$ aus $NO_3$ | − | L-Histidin | + |
| Denitrifikation | − | Hydroxybutyrat | + |

EP 0 502 525 B1

```
Phenylalanindesaminase          -          N-Quelle

Levan aus Saccharose            -          NH4+                    ++

Lecithinase                     -          R,S-(±)-2,2-Dimethyl-

Urease                          -          cyclopropanacarboxamid +
```

Wissenschaftliche Beschreibung von <u>Bacterium</u> <u>sp.</u> VIII:II (DSM-Nr.6316)

| | |
|---|---|
| Gram Färbung | + |
| Gram Reaktion (KOH Test) | - |
| Oxidase | - |
| Catalase | - |
| Nitrat Reduktion | - |
| Truptophan → Indol | - |
| Glucose (anaerob) | - |
| Arginin | - |
| Urease | - |
| Eskulin | + |
| Gelatin | - |
| β-Galactosidase | + |
| Glucose | + |
| Arabinose | - |
| Mannose | (+) |
| Mannit | + |
| N-Acetylglucosamin | - |
| Maltose | + |
| Gluconat | - |
| Caprat | - |
| Adipat | - |
| Malat | - |
| Citrat | - |
| Phenylacetat | - |

<u>Vorkultur</u>

Üblicherweise wird nach dem Selektionsverfahren eine Vorkultur dieser Mikroorganismen angelegt, mit der dann auch weitere Kulturen beimpft werden können.

Diese Vorkultur kann in den gleichen Medien, mit dem gleichen C/N-Verhältnis und mit den gleichen Verbindungen als C-Quelle analog zum Selektionsverfahren gezüchtet werden.

Vorzugsweise enthält das Medium für die Vorkultur entweder die Zusammensetzung, die in Tabelle 2 oder die, die in Tabelle 3 mit oder ohne Universalpepton angegeben ist.

Als N-Quelle können für die Vorkultur, die in der Fachwelt üblichen, angewendet werden, vorzugsweise wird als N-Quelle ein Ammoniumsalz, wie beispielsweise Ammoniumsulfat, angewendet.

Der pH-Wert in der Vorkultur liegt zweckmässig zwischen pH 4 und 10 bei einer zweckmässigen Temperatur von 20 bis 40°C.

Nach einer Kultivierungsdauer von in der Regel 10 bis 100 h können die Mikroorganismen induziert und für die Biotransformation vorbereitet werden.

<u>Induktion</u>

Üblicherweise wird mit dieser Vorkultur das Induktions-Medium beimpft, welches bis auf die N-Quelle die gleiche Zusammensetzung hat wie das Vorkultur-Medium.

Zweckmässig enthält dieses Medium zur Induktion der wirksamen Enzyme der Mikroorganismen 2,2-DMCPCA in

7

Form des Racemats oder seiner optischen Isomere, das sowohl als N-Quelle als auch als Enzym-Induktor dienen kann.

Denkbar sind jedoch auch andere Verbindungen als Induktoren, wie beispielsweise Cyclopropancarboxamid, Caprolactam, Benzamid, Cyclohexancarboxamid, Nikotinsäureamid oder Crotonamid. Die Induktion erfolgt zweckmässig mit 0,05 bis 0,15 Gew.%, vorzugsweise mit 0,1 bis 0,15 Gew.%,2,2-DMCPCA in Form des Racemats oder seiner optischen Isomere.

Nach einer üblichen Induktionsdauer von 15 bis 80 h können die Mikroorganismen beispielsweise durch Zentrifugieren oder Ultrafiltration geerntet und dann in einem Medium für das Verfahren resuspendiert werden.

Biotransformation

Das eigentliche Verfahren zur Herstellung von S-(+)-2,2-DMCPCA und R-(-)-2,2-DMCPCS wird erfindungsgemäss derart durchgeführt, dass man im racemischen R,S-(±)-2,2-DMCPCA das R-(-)- 2,2-DMCPCA zur R-(-)-2,2-DMCPCS biotransformiert, wobei neben R-(-)-2,2-DMCPCS optisch aktives S-(+)-2,2-DMCPCA anfällt und die Produkte getrennt isoliert werden.

Racemisches R,S-(±)-2,2-DMCPCA kann beispielsweise chemisch oder enzymatisch ausgehend von R,S-(±)-2,2-Dimethylcyclopropancarbonsäurenitril hergestellt werden.

Zweckmässig wird die Biotransformation entweder mit Mikroorganismen durchgeführt oder mit Enzymen in einem zellfreien System.

Die Enzyme für das zellfreie System können durch fachmännisch übliches Aufschliessen der Mikroorganismenzellen gewonnen werden. Hierzu kann beispielsweise die Ultraschall-, French-Press- oder Lysozym-Methode angewendet werden.

Diese zellfreien Enzyme können dann beispielsweise für die Durchführung des Verfahrens auf einem geeigneten Trägermaterial immobilisiert werden.

Für das Verfahren besonders geeignet sind die Mikroorganismen der Spezies <u>Comamonas acidovorans</u> A:18 (DSM-Nr.6315), <u>Comamonas acidovorans</u> TG 308 (DSM-Nr.6552), <u>Pseudomonas sp</u> NSAK:42 (DSM-Nr.6433) und der Spezies <u>Bacterium sp.</u> VIII:II (DSM-Nr. 6316), deren Deszendenten und Mutanten sowie andere Mikroorganismen, die nach dem hier beschriebenen Verfahren selektiert werden. Ebenso geeignet sind die zellfreien Enzyme aus diesen Mikroorganismen.

Vorzugsweise wird das Verfahren entweder mit ruhenden Mikroorganismenzellen (nicht wachsende Zellen), die keine C- und N-Quelle mehr benötigen, durchgeführt oder mit Enzymen in einem zellfreien System, wobei diese Enzyme wie zuvor beschrieben, beispielsweise durch Aufschliessen der induzierten Mikroorganismen,erhalten werden.

Zweckmässig enthält das Medium das racemische 2,2-DMCPCA in einer Menge von 0,2 bis 5 Gew.%, vorzugsweise von 0,2 bis 2 Gew.%.

Als Medium für das Verfahren können die in der Fachwelt üblichen dienen, wie beispielsweise niedermolare Phosphat-Puffer, das zuvor beschriebene Mineralsalzmedium oder auch HEPES-Puffer (N-2-Hydroxyethylpiperazin-2'-ethansulfonsäure).

Vorzugsweise wird das Verfahren in einem niedermolaren Phosphat-Puffer durchgeführt.

Der pH-Wert des Mediums kann in einem Bereich von pH 6 bis 11 liegen, vorzugsweise liegt der pH-Wert zwischen 6,5 und 10.

Zweckmässig wird die Biotransformation bei einer Temperatur von 15 bis 55°C durchgeführt, vorzugsweise bei 20 bis 40°C.

Nach einer üblichen Umsetzungsdauer von 1 bis 30 h, vorzugsweise von 5 bis 25 h, ist das R-(-)-2,2-DMCPCA vollständig zur entsprechenden Säure umgewandelt, wobei auch optisch reines S-(+)-2,2-DMCPCA anfällt.

Das so gewonnene S-(+)-2,2-DMCPCA kann dann beispielsweise durch Extraktion, Elektrodialyse oder durch Trocknung gewonnen werden.

Beispiel 1

Isolation und Selektion von Mikroorganismen mit einer R-(-)-2,2-DMCPCA-Hydrolase

Zu zerkleinerten Proben aus dem Erdreich (1g) wurde isotonische Kochsalzlösung (9 ml) hinzugegeben und das Ganze für ca. 5 min stehen gelassen. Danach wurde der Überstand (0,5 ml) in ein Mineralsalzmedium (25 ml) (Kulla et al., Arch. Microbiol. 135, S.1-7, 1983) überimpft, welches Glycerin und R,S-(±)-DMCPCA in einem C-/N-Verhältnis von 5:1 enthielt. Anschliessend wurde diese Kultur inkubiert, bis eine Mischkultur entstanden war, die R,S-(±)-2,2-DMCPCA als N-Quelle benutzen kann.

Reinkulturen dieser Mikroorganismen wurden unter Zuhilfenahme traditioneller mikrobiologischer Techniken erhalten.

Die auf diese Weise erhaltenen Mikroorganismenstämme wurden dann auf Agar-Platten für ihr Wachstum auf R,S-(±)-2,2-DMCPCA, S-(+)-2,2-DMCPCA und R-(-)-2,2-DMCPCA getestet, um die nicht gewünschten Stämme herauszufinden, die auf beiden Isomeren (R-(-)-2,2-DMCPCA und S-(+)-2,2-DMCPCA) gleich schnell wachsen.

Die übrigen Stämme wurden weiter getestet. Mit diesen wurde dann ein Vorkultur-Medium angeimpft. Die in dieser Vorkultur erhaltenen Mikroorganismen wurden ins Mineralsalzmedium überführt und dann auf ihre Fähigkeit überprüft, selektiv R-(-)-2,2-DMCPCA als einzige N-Quelle zu nutzen, wobei der Überstand mittels GC auf die Bildung von R-(-)-2,2-DMCPCS und auf die Anreicherung von S-(+)-2,2-DMCPCA überprüft wurde.

Beispiel 2

Aktivitätsbestimmung der R-(-)-2,2-DMCPCA-Hydrolase

Zur Aktivitätsbestimmung der Hydrolase wurde die Mikroorganismensuspension auf eine optische Dichte von 0,5 bei 650 nm eingestellt. Als Medium diente ein Phosphat-Puffer (10 mmolar), pH 7,0, mit 0,2 Gew.% R,S-2,2-DMCPCA. Diese Suspension wurde 3 h bei 30°C unter Schütteln inkubiert. Das durch die Hydrolase freigesetzte $NH_4^+$ oder die R-(-)-2,2-DMCPCS wurde gemessen und die Aktivität als g R-(-)-2,2-DMCPCA umgesetzt /l/h/optische Dichte bei 650 nm ausgedrückt, vorausgesetzt, dass 1 mmol gebildetes $NH_4^+$ = 1 mmol umgesetztem R-(-)-2,2-DMCPCA entspricht.

Tabelle 1

| Bestimmung der Hydrolase-Aktivität in Abhängigkeit von der Temperatur (Stamm Comamonas acidovorans A:18, DSM-Nr.6315) | |
|---|---|
| Temperatur [°C] | Aktivität g/l/h/$OD_{650}$ |
| 25 | 0,25 |
| 30 | 0,5 |
| 37 | 1,0 |
| 45 | 1,5 |
| 48 | 1,8 |
| 55 | 1,9 |
| 65 | 0 |

Beispiel 3

Herstellung von S-(+)-2,2-DMCPCA und R-(-)-2,2-DMCPCS

Comamonas acidovorans A:18 wurde auf Mineralsalzmedium-Agar-Platten, mit Glycerin als C-Quelle und Ammoniumsulfat als N-Quelle 2 Tage lang bei 30°C bebrütet. Die Zusammensetzung dieses Mineralsalzmediums ist in Tabelle 2 angegeben. Mit diesen ausplattierten Mikroorganismen wurde ein Vorkultur-Medium mit der gleichen Zusammensetzung beimpft und 2 Tage lang bei 30°C inkubiert.

Das gleiche Mineralsalzmedium mit $Na_2SO_4$ anstatt $(NH_4)_2SO_4$ (100 ml), enthaltend 0,2 Gew.% Glycerin und 0,15 Gew.% R,S-(±)-2,2-DMCPCA, wurde mit 5 ml dieser Vorkultur zur Induktion beimpft und bei 30°C 45 h lang inkubiert. Anschliessend wurden die Zellen durch Zentrifugation geerntet und in 0,9%iger NaCl-Lösung aufgenommen.

Nach Resuspension der Zellen in 10 mmolarem Phosphat-Puffer (800 ml), pH 7,0, wurde eine optische Dichte bei 650 nm von 1,3 eingestellt und 2 Gew.% R,S-(±)-2,2-DMCPCA zugefügt. Nach einer Inkubation von ca. 25 h bei 37°C wurde R-(-)-2,2-DMCPCA vollständig zur R-(-)-2,2-DMCPCS umgesetzt, was einer optischen Reinheit (ee) von 100% und einer analytisch gemessenen Ausbeute an S-(+)-2,2-DMCPCA von 46,7% entsprach.

Der Reaktionsablauf wurde anhand der $NH_4^+$-Freisetzung und anhand von GC-Analyse des Überstandes verfolgt.

Nach dem Abzentrifugieren der Zellen wurde der Überstand auf einen pH-Wert von 9,5 eingestellt und S-(+)-2,2-DMCPCA durch Extraktion mit Ethylacetat isoliert.

Beispiel 4

In analoger Weise zu Beispiel 1 wurde der Mikroorganismus Bacterium sp. VIII:II isoliert.

Die Vorkultivierungsdauer betrug bei diesem Mikroorganismus 4 Tage und die Induktionsdauer 3 Tage unter den ansonsten gleichen Bedingungen wie Beispiel 3.

Im Gegensatz zu Beispiel 3 wurde die Biotransformation bei diesem Mikroorganismus mit 0,2 Gew.% R,S-(±)-2,2-DMCPCA durchgeführt, wobei die Aktivität der Hydrolase zu 0,13 g/l/h/ $OD_{650nm}$ bestimmt wurde.

Tabelle 2

| Medium-Zusammensetzung der Vorkultur (Kulla et al., Arch. Microbiol. 135, S. 1-7, 1983) | |
|---|---|
| g/l destilliertes Wasser pH 7,0 | |
| 2,0 | $(NH_4)_2 SO_4$ |
| 2,5 | $Na_2HPO_4 \cdot 2H_2O$ |
| 1,0 | $KH_2PO_4$ |
| 3,0 | NaCl |
| 0,4 | $MgCl_2 \cdot 6H_2O$ |
| 0,015 | $CaCl_2 \cdot 2H_2O$ |
| 0,0008 | $FeCl_2 \cdot 6H_2O$ |
| 0,0001 | $ZnSO_4 \cdot 7H_2O$ |
| 0,00009 | $MnCl_2 \cdot 4H_2O$ |
| 0,0003 | $H_3BO_3$ |
| 0,0002 | $CoCl_2 \cdot 6H_2O$ |
| 0,00001 | $CuCl_2 \cdot 2H_2O$ |
| 0,00002 | $NiCl_2 \cdot 6H_2O$ |
| 0,00003 | $Na_2MoO_4 \cdot 2H_2O$ |
| 0,005 | $Na_2EDTA \cdot 2H_2O$ |
| 0,002 | $FeSO_4 \cdot 7H_2O$ |
| 2 | Glycerin |

Beispiel 5

In analoger Weise zu Beispiel 1 wurde Pseudomonas sp NSAK:42 (DSM-Nr.6433) isoliert. Die Vorkultivierungsdauer betrug 2 Tage und die Induktionsdauer 18 h unter den ansonsten gleichen Bedingungen wie Beispiel 3, mit Ausnahme, dass das Induktionsmedium 1 Gew.% Glycerin (anstatt 0,2 Gew.%) und zusätzlich noch 0,3 Gew.% Universalpepton (Merck) enthielt. Die Biotransformation wurde mit 0,2 Gew.% R,S-(±)-2,2-DMCPCA durchgeführt, wobei die Aktivität der Hydrolase zu 0,34 g/l/h/$OD_{650nm}$ bestimmt wurde.

Beispiel 6

Comamonas acidovorans TG 308 (DSM-Nr.6552) wurde für 2 Tage bei 30°C im Komplexmedium (4 ml) "Nutrient Broth" (Oxoid Ltd, GB) kultiviert. Mit diesen Mikroorganismen wurde ein Mineralsalzmedium (Tabelle 3) beimpft und 2 Tage lang bei 30°C inkubiert.

Anschliessend wurden die Zellen entsprechend zu Beispiel 3 geerntet.

Im Gegensatz zu Beispiel 3 wurde die Biotransformation in 10 mmolarem HEPES-Puffer (pH 7,0) mit 0,5 Gew.% R,S-(±)-2,2-DMCPCA unter den sonst gleichen Bedingungen wie in Beispiel 3 durchgeführt.

Nach einer Inkubation von 4,5 h bei 37°C wurde R-(-)-2,2-DMCPCA vollständig zur R-(-)-2,2-DMCPCS umgesetzt, was einer analytisch gemessenen Ausbeute von S-(+)-DMCPCA von 45,5% und einer optischen Reinheit (ee) von 99,0% entsprach.

Tabelle 3

| g/l destilliertes Wasser pH 7,0 | |
|---|---|
| 2,0 | $K_2 HPO_4$ |
| 2,0 | $KH_2PO_4$ |
| 2,0 | $MgSO_4 \cdot 7H_2O$ |
| 0,5 | Hefe-Extrakt |
| 2,0 | Universalpepton (Merck) |
| 2,0 | NaCl |
| 0,01 | $FeCl_2 \cdot H_2O$ |
| 10 | Natriumglutamat |
| 5 | Crotonamid |

Beispiel 7

Herstellung von S-(+)-2,2-DMCPCA und R-(-)-2,2-DMCPCS mit Enzymen in einem zellfreien System

Nach Induktion wurden die Zellen von Comamonas acidovorans A:18 auf eine optische Dichte bei 650 nm von 210 auf 95 ml im HEPES-Puffer (10 mM, pH 7,0) konzentriert. Anschliessend wurden die Zellen 2 mal in der French-Press bei einem Druck von 1200 bar aufgeschlossen. Um einen zellfreien Enzymextrakt zu erhalten, wurde dann das Ganze bei 20 000 rpm für 20 min zentrifugiert.

In diesem zellfreien Enzymextrakt wurde dann die Proteinmenge (gemessen nach der Bradford-Methode) zu 39,3 mg Protein/ml bestimmt.

Für die Aktivitätsbestimmung dieser zellfreien Enzyme wurden 20 µl (ca. 0,8 mg Protein) dieses zellfreien Enzym-Extrakts in 4 ml Phosphat-Puffer (10 mM, pH 7,0), enthaltend 0,2 Gew.% R,S-(±)-2,2-DMCPCA, aufgenommen und bei 30°C inkubiert. Dabei wurden im zellfreien Extrakt 2,5 g R-(-)-2,2-DMCPCA/h/g Protein (3,64 µmole/min/g Protein) zur entsprechenden Säure umgewandelt.

Beispiel 8

Herstellung von S-(+)-2,2-DMCPCA und R-(-)-2,2-DMCPCS mit immobilisierten Zellen

Nach Induktion (entsprechend zu Beispiel 3) wurden die Zellen von Comamonas acidovorans A:18 abgeerntet.

Diese Zellen wurden durch eine Polyethylenimin/Glutaraldehyd-Behandlung (entsprechend der CS-PS 251 111) immobilisiert. Der immobilisierte Biokatalysator hatte eine Aktivität von 1,6 µmol/min·g Trockengewicht. Das Trockengewicht des Biokatalysators entsprach 32% des Feuchtgewichtes.

Dann wurde der Biokatalysator (mit 55 g Feuchtgewicht) in 800 ml Borsäure (10 mM, pH 9,0) mit 16 g R,S-(±)-2,2-DMCPCA suspendiert.

Nach einer Inkubationszeit von 68,8 h bei 37°C und bei einer Rührgeschwindigkeit von 200 rpm wurde R-(-)-2,2-DMCPCA vollständig zur R-(-)-2,2-DMCPCS umgesetzt, was einer optischen Reinheit (ee) von 98,2% und einer analytisch gemessenen Ausbeute an S-(+)-2,2-DMCPCA von 40% entsprach.

**Patentansprüche**

1. Verfahren zur Herstellung von S-(+)-2,2-Dimethylcyclopropancarboxamid und/oder R-(-)-2,2-Dimethylcyclopropancarbonsäure, dadurch gekennzeichnet, daß man im racemischen R,S-(±)-2,2-Dimethylcyclopropancarboxamid das R-(-)-2,2-Dimethylcyclopropancarboxamid zur R-(-)-2,2-Dimethylcyclopropancarbonsäure biotransformiert und gegebenenfalls isoliert, wobei bei der Biotransformation neben R-(-)-2,2-Dimethylcyclopropancarbonsäure optisch aktives S-(+)-2,2-Dimethylcyclopropancarboxamid anfällt, das gegebenenfalls isoliert wird.

2. Verfahren nach Patentanspruch 1, dadurch gekennzeichnet, daß man die Biotransformation mit Mikroorganismen durchführt.

3. Verfahren nach mindestens einem der Patentansprüche 1 und 2, dadurch gekennzeichnet, daß man die Biotransformation mit Enzymen in einem zellfreien System durchführt.

4. Verfahren nach mindestens einem der Patentansprüche 1 bis 3, dadurch gekennzeichnet, daß man die Biotransformation in einem Medium, enthaltend racemisches 2,2-Dimethylcyclopropancarboxamid in einer Menge vonm 0,2 bis 5 Gew.%, durchführt.

5. Verfahren nach mindestens einem der Patentansprüche 1 bis 4, dadurch gekennzeichnet, daß man die Biotransformation bei einem pH-Wert von 6 bis 11 und bei einer Temperatur von 15 bis 55°C durchführt.

6. Verfahren nach mindestens einem der Patentansprüche 1 bis 5, dadurch gekennzeichnet, daß man die Biotransformation entweder mit Mikroorganismen der Spezies Comamonas acidovorans A:18 (DSM-Nr.6315) oder deren Deszendenten und Mutanten oder mit zellfreien Enzymen aus diesen Mikroorganismen durchführt.

7. Verfahren nach mindestens einem der Patentansprüche 1 bis 5, dadurch gekennzeichnet, daß man die Biotransformation entweder mit Mikroorganismen der Spezies Comamonas acidovorans TG 308 (DSM-Nr.6552) oder deren Deszendenten und Mutanten oder mit zellfreien Enzymen aus diesen Mikroorganismen durchführt.

**8.** Verfahren nach mindestens einem der Patentansprüche 1 bis 5, dadurch gekennzeichnet, daß man die Biotransformation entweder mit Mikroorganismen der Spezies <u>Pseudomonas sp</u> NSAK:42 (DSM-Nr.6433) oder deren Deszendenten und Mutanten oder mit zellfreien Enzymen aus diesen Mikroorganismen durchführt.

**9.** Verfahren nach mindestens einem der Patentansprüche 1 bis 5, dadurch gekennzeichnet, daß man die Biotransformation entweder mit Mikroorganismen der Spezies <u>Bacterium sp.</u> VIII:II (DSM-Nr.6316) oder deren Deszendenten und Mutanten oder mit zellfreien Enzymen aus diesen Mikroorganismen durchführt.

**10.** Mikroorganismen, die befähigt sind, im racemischen R,S-(±)-2,2-Dimethylcyclopropancarboxamid das R-(-)-2,2-Dimethylcyclopropancarboxamid zur R-(-)-2,2-Dimethylcyclopropancarbonsäure zu biotransformieren, wobei neben R-(-)-2,2-Dimethylcyclopropancarbonsäure optisch aktives S-(+)-2,2-Dimethylcyclopropancarboxamid anfällt.

**11.** Mikroorganismen nach Patentanspruch 10, erhältlich nach folgender Selektionsmethode:

a) Mikroorganismen, die mit 2,2-Dimethylcyclopropancarboxamid, in Form des Racemats oder seiner optischen Isomere als N-Quelle und mit einer C-Quelle wachsen, werden gezüchtet;
b) aus der durch Züchtung erhaltenen Kultur werden dann jene selektioniert, die stabil sind und 2,2-Dimethylcyclopropancarboxamid, in Form des Racemats oder seiner optischen Isomere, als einzige N-Quelle nutzen.

**12.** Mikroorganismen nach mindestens einem der Patentansprüche 10 und 11, dadurch gekennzeichnet, daß sie als C-Quelle Zucker, Zuckeralkohole, Carbonsäuren, Alkohole oder andere C-Quellen als Wachstumssubstrat nutzen.

**13.** Mikroorganismen nach mindestens einem der Patentansprüche 10 bis 12, dadurch gekennzeichnet, daß sie als N-Quelle R-(-)-2,2-Dimethylcyclopropancarboxamid akzeptieren.

**14.** Mikroorganismus <u>Comamonas acidovorans</u> A:18 (DSM-Nr.6315) und dessen Deszendenten und Mutanten.

**15.** Mikroorganismus <u>Comamonas acidovorans</u> TG 308 (DSM-Nr. 6552) und dessen Deszendenten und Mutanten.

**16.** Mikroorganismus <u>Pseudomonas sp</u> NSAk:42 (DSM-Nr. 6433) und dessen Deszendenten und Mutanten.

**17.** Mikroorganismus <u>Bacterium sp.</u> VIII:II (DSM-Nr.6316) und dessen Deszendenten und Mutanten.

## Claims

**1.** A process for the production of S-(+)-2,2-dimethylcyclopropanecarboxamide and/or R-(-)-2,2-dimethylcyclopropane carboxylic acid, characterized in that the R-(-)-2,2-dimethylcyclopropanecarboxamide in racemic R,S-(±)-2,2-dimethylcyclopropanecarboxamide is biotransformed to R-(-)-2,2-dimethylcyclopropane carboxylic acid which is optionally isolated, in which biotransformation besides R-(-)-2,2-dimethylcyclopropane carboxylic acid the optically active S-(+)-2,2-dimethylcyclopropanecarboxamide is obtained which is optionally isolated.

**2.** The process according to claim 1, characterized in that the biotransformation is performed with microorganisms.

**3.** The process according to at least one of the claims 1 and 2, characterized in that the biotransformation is performed with enzymes in a cell-free system.

**4.** The process according to at least one of the claims 1 to 3, characterized in that the biotransformation is performed in a medium containing racemic 2,2-dimethylcyclopropanecarboxamide in an amount of 0.2 to 5 percent by weight.

**5.** The process according to at least one of the claims 1 to 4, characterized in that the biotransformation is performed at a pH value of 6 to 11 and at a temperature of 15 to 55 °C.

**6.** The process according to at least one of the claims 1 to 5, characterized in that the biotransformation is performed either with microorganisms of the species <u>Comamonas acidovorans</u> A:18 (DSM No. 6315), or with descendants or mutants thereof, or with cell-free enzymes from these microorganisms.

7. The process according to at least one of the claims 1 to 5, characterized in that the biotransformation is performed either with microorganisms of the species <u>Comamonas acidovorans</u> TG 308 (DSM No. 6552), or with descendants or mutants thereof, or with cell-free enzymes from these microorganisms.

8. The process according to at least one of the claims 1 to 5, characterized in that the biotransformation is performed either with microorganisms of the species <u>Pseudomonas sp</u> NSAK:42 (DSM No. 6433), or with descendants or mutants thereof, or with cell-free enzymes from these microorganisms.

9. The process according to at least one of the claims 1 to 5, characterized in that the biotransformation is performed either with microorganisms of the species <u>Bacterium sp.</u> VIII:II (DSM No. 6316), or with descendants or mutants thereof, or with cell-free enzymes from these microorganisms.

10. Microorganisms capable of biotransforming the R-(-)-2,2-dimethylcyclopropanecarboxamide in racemic R,S-(±)-2,2-dimethylcyclopropanecarboxamide to R-(-)-2,2-dimethylcyclopropane carboxylic acid, wherein besides R-(-)-2,2-dimethylcyclopropane carboxylic acid the optically active S-(+)-2,2-dimethylcyclopropanecarboxamide is obtained.

11. The microorganisms according to claim 10, obtainable according to the following method of selection:

    a) microorganisms, which grow with 2,2-dimethylcyclopropanecarboxamide, in the form of the racemate or its optical isomers, as an N-source together with a C-source, are cultivated; and
    b) from the culture obtained by cultivation, those microorganisms are then selected which are stable and use 2,2-dimethylcyclopropanecarboxamide, in the form of the racemate or its optical isomers, as the sole N-source.

12. The microorganisms according to at least one of the claims 10 and 11, characterized in that they use as a C-source sugars, sugar alcohols, carboxylic acids, alcohols or other C-sources as growth substrate.

13. The microorganisms according to at least one of the claims 10 to 12, characterized in that they accept R-(-)-2,2-dimethylcyclopropanecarboxamide as an N-source.

14. The microorganism <u>Comamonas acidovorans</u> A:18 (DSM No. 6315) and the descendants and mutants thereof.

15. The microorganism <u>Comamonas acidovorans</u> TG 308 (DSM No. 6552) and the descendants and mutants thereof.

16. The microorganism <u>Pseudomonas sp</u> NSAK:42 (DSM No. 6433) and the descendants and mutants thereof.

17. The microorganism <u>Bacterium sp.</u> VIII:II (DSM No. 6316) and the descendants and mutants thereof.

**Revendications**

1. Procédé pour la préparation de S-(+)-2,2-diméthylcyclopropanecarboxamide et/ou de l'acide R-(-)-2,2-diméthylcyclopropanecarboxylique, caractérisé en ce que l'on biotransforme et éventuellement que l'on isole dans le R,S-(±)-2,2-diméthylcyclopropanecarboxamide racémique en acide R-(-)-2,2-diméthylcyclopropanecarboxylique, moyennant quoi au cours de la biotransformation en plus de l'acide R-(-)-2,2-diméthylcyclopropanecarboxylique il est produit du S-(±)-2,2-diméthylcyclopropanecarboxamide actif optiquement, qui est éventuellement isolé.

2. Procédé selon la revendication 1, caractérisé par ce que l'on réalise la biotransformation avec des microorganismes.

3. Procédé selon au moins l'une des revendications 1 et 2, caractérisé par ce que l'on effectue la biotransformation avec des enzymes dans un système acellulaire.

4. Procédé selon au moins l'une des revendications 1 à 3, caractérisé par ce que l'on effectue la biotransformation dans un milieu contenant le 2,2-diméthylcyclopropanecarboxamide racémique dans une quantité de 0,2 à 5 % en poids.

5. Procédé selon au moins l'une des revendications 1 à 4, caractérisé par ce que l'on effectue la biotransformation à un pH de 6 à 11 et à une température de 15 à 55°C.

6.  Procédé selon au moins l'une des revendications 1 à 5, caractérisé en ce que l'on effectue la biotransformation soit avec des microorganismes de l'espèce <u>Comamonas acidovorans</u> A:18 (DSM-Nr.6315) soit leurs descendants et mutants ou avec des enzymes acellulaires à partir de ces microorganismes.

7.  Procédé selon au moins l'une des revendications 1 à 5, caractérisé en ce que l'on effectue la biotransformation soit avec des microorganismes de l'espèce <u>Comamonas acidovorans</u> TG 308 (DSM-Nr.6552), soit avec leurs descendants et mutants ou avec des enzymes acellulaires à partir de ces microorganismes.

8.  Procédé selon au moins l'une des revendications 1 à 5, caractérisé en ce que l'on effectue la biotransformation soit avec des microorganismes de l'espèce <u>Pseudomonas sp</u> NSAK:42 (DSM-Nr.6433) soit avec leurs descendants et mutants ou avec des enzymes acellulaires à partir de ces microorganismes.

9.  Procédé selon au moins l'une des revendications 1 à 5, caractérisé en ce que la biotransformation s'effectue soit avec des microorganismes de l'espèce <u>Bacterium sp.</u> VIII:II (DSM-Nr.6316) soit avec leurs descendants et mutants ou avec des enzymes acellulaires à partir de ces microorganismes.

10. Microorganismes qui sont aptes à biotransformer, dans le R,S-(±)-2,2-diméthylcyclopropanecarboxamide racémique, le R-(-)-2,2-diméthylcyclopropanecarboxamide en l'acide R-(-)-2,2-diméthylcyclopropanecarboxylique, ce faisant en plus de l'acide R-(-)-2,2-diméthylcyclopropanecarboxylique, du S-(+)-2,2-diméthylcyclopropanecarboxamide actif optiquement étant produit.

11. Microorganismes selon la revendication 10, pouvant être obtenus selon le mode de sélection suivant :

    a) microorganismes qui sont sélectionnés avec le 2,2-diméthylcyclopropanecarboxamide sous forme du racémate ou de ses isomères optiques en tant que source N et qui croissent avec une source C ;
    b) à partir de la culture obtenue par sélection, on sélectionne alors les microorganismes qui sont stables et le 2,2-diméthylcyclopropanecarboxamide, sous forme du racémate ou de ses isomères optiques en tant que source N unique.

12. Microorganismes selon au moins l'une des revendications 10 et 11, caractérisés en ce qu'ils utilisent en tant que source C du sucre, des alcools de sucre, des acides carboxyliques, des alcools ou d'autres sources C en tant que substrat de croissance.

13. Microorganismes selon une des revendications 10 à 12 caractérisés en ce qu'ils acceptent en tant que source N le R-(-)2,2-diméthylcyclopropanecarboxamide.

14. Microorganisme <u>Comamonas acidovorans</u> A:18 (DSM-Nr.6315) et ses descendants et mutants.

15. Microorganisme <u>Comamonas acidovorans</u> TG 308 (DSM-Nr.6552) et ses descendants et mutants.

16. Microorganisme <u>Pseudomonas sp</u> NSAK:42 (DSM-Nr.6433) et ses descendants et mutants.

17. Microorganisme <u>Bacterium sp</u> VIII:II (DSM-Nr.6316) et ses descendants et mutants.